# EUROPÄISCHE PATENTANMELDUNG

(11) **EP 2 767 831 A1**
(43) Veröffentlichungstag der Anmeldung: **20.08.2014**
(21) Anmeldenummer: 13155867.8
(22) Anmeldetag: 19.02.2013
(51) Int. Cl.: G01N 33/543, B01L 3/00, G01N 33/558

(54) **Neues PoC-Testsystem und Verfahren**

(71) Anmelder: DST Diagnostische Systeme & Technologien GmbH, 19059 Schwerin (DE)
(72) Erfinder: Dangers, Marc, 19053 Schwerin (DE); Rübenhagen, René, 19061 Schwerin (DE)
(74) Vertreter: Simandi, Claus

(57) **Zusammenfassung**

Testverfahren zum Nachweis von mindestens einem Analyten aus einer Probenflüssigkeit umfassend zwei Probenkammern, die durch einen Träger mit mindestens zwei Oberflächen getrennt sind, wobei eine Vorderseite des Trägers zur ersten Probenkammer und eine Rückseite des Trägers zur zweiten Probenkammer weist vorzugsweise für den Gebrauch im Point-of-Care (PoC) Bereich.

## Beschreibung

Die vorliegende Erfindung betrifft ein Testsystem oder Assaysystem (Nachweissystem)und Testverfahren vorzugsweise im Gebrauch für den Point-of-Care (PoC) Bereich.

In der Forschung und in-vitro Diagnostik im humanen, veterinären, Lebensmittel- oder in einer Vielzahl weiterer Anwendungsbereiche dienen analytische Tests zur qualitativen oder/und quantitativen Bestimmung von Molekülen, Analyten bzw. deren Aktivität oder Zusammensetzung. Die Testresultate liefern unterschiedliche Aussagen wie beispielsweise diagnostische Parameter zur Identifikation von Krankheiten, Herkunft von Lebensmitteln oder Wirksamkeit eines Medikaments.

Heute dominieren die bekannten Methoden der DNA- und Proteinanalytik in den verfügbaren Tests. Insbesondere Immunoassays stellen Verfahren dar, bei denen Antikörper zur spezifischen Bindung von ausgesuchten, ausreichend großen Zielmolekülen fast beliebiger Art, beispielsweise Biomarkern, eingesetzt werden.

Schnelltests liefern Messwerte innerhalb von wenigen Minuten oder Stunden nach Beginn des Tests, und nahe am Ort der Probennahme und ohne Notwendigkeit, die Probe einzusenden (Point-of-Care (PoC)).

Bekannte standardisierte Testverfahren sind Lateral-Flow-Test (LFT), Flow-Through-Test (FTT), Agglutinations-Test (AT) oder Solid-Phase-Test (SPT). Alle diese Verfahren weisen Analyte direkt visuell nach. Für solche Tests gibt es zusätzlich kompakte Auslesegeräte, die auch quantitative Ergebnisse liefern.

Bekannte Assayverfahren der in-vitro Diagnostik sind Immunoassays (IA), insbesondere Enzyme-linked Immunoassays (EIA), oder "binding assay" ("sandwich") (Siehe z.B. EP0171150B1, EP 0063810B1). Darüber hinaus sei auf das Schrifttum von Roger P. Ekins (z.B. WO 8401031 u.v.a.) verwiesen.

Membrangestütze Bindungsassays, insbesondere von IgE aus Blut an Allergenen sind seit Ende der 1980-iger Jahre bekannt, so etwa aus CHEMICAL ABSTRACTS, vol. 101, no. 25, 17th December 1984, page 578, abstract no. 228190b, Columbus, Ohio, US; B.J. WALSH et al.: "Allergen discs prepared from nitrocellulose: detection of IgE binding to soluble and insoluble allergens", & J. IMMUNOL. METHODS 1984, 73(1), 139-45. Einige Testsysteme, die auf dem Prinzip des Lateral Flow beruhen, sind als Allergieschnelltest kommerziell erhältlich.

Für den PoC - Bereich ist beispielsweise der käuflich erhältliche Fast-Check-PoC der Anmelderin beschrieben (siehe EP1718970), der auf Basis eines membrangestützten Bindungsassays zum Allergienachweis aus Vollblut eingesetzt werden kann.

Ferner ist ein Schnelltest der Anmelderin in WO2011/000959 beschrieben, bei dem eine Membran gezielt zwischen den Nachweisstellen benetzt wird, um die Testreagenzien besonders effektiv und parallel in spezifische Regionen der zuvor mit dem zu analysierenden Vollblut beladene Membran einzubringen.

Ein weiterer Schnelltest der Anmelderin ist in PCT/EP2012/066155 (unveröffentlicht) beschrieben, bei dem eine

Membran von beiden Seiten umspült wird. Hierzu ist die Membran an den Längsseiten in einer Vorrichtung fixiert, so dass an ihrer Ober- und Unterseite ein Freivolumen entsteht, durch das Flüssigkeiten an der Membran vorbeifließen können.

Es besteht jedoch ein großer Bedarf an verbesserten Schnelltests.

Insbesondere die gleichmäßige Beladung der einzelnen Nachweisplätze mit Probenflüssigkeit (Analyt) ist verbesserungsfähig, als auch eine effiziente Spülung aller Nachweisplätze, sowie eine reproduzierbare Strömungsleitung der Flüssigkeiten, die die durch Fertigungstoleranzen bedingte Variationen der Membraneigenschaften toleriert. All diese Eigenschaften sind jedoch Voraussetzung für reproduzierbare und qualitativ hochwertige Ergebnisse.

Daher ist es Aufgabe der vorliegenden Erfindung für ein PoC-Testsystem ein verbessertes und besser reproduzierbares Verfahren zum Beladen eines Trägers mit Probenflüssigkeit zum Nachweis von Analyten mittels Rezeptormolekülen bereitzustellen.

Überraschender Weise wird diese Aufgabe durch den Anspruch 1 gelöst. Erfindungsgemäß ist ein Testverfahren zum Nachweis mindestens einem Analyten aus einer Probenflüssigkeit vorgesehen,
bestehend aus zwei Probenkammern, die vorzugsweise durch einen für eine Probenflüssigkeit teilweise durchlässigen Träger bestehend aus mindestens zwei Oberflächen getrennt werden, wobei eine Vorderseite des Trägers zur ersten Probenkammer und eine Rückseite des Trägers zur zweiten Probenkammer weist, und
a) mindestens ein Rezeptormolekül auf der Vorderseite eines Trägers fixiert ist,
b) über der Vorder- und Rückseite des Trägers jeweils zumindest ein Freivolumen ausgebildet ist, welches von einer Kammerwand begrenzt ist,
c) die erste Probenkammer mindestens zwei Öffnungen aufweist, wobei durch eine erste Öffnung eine Probenflüssigkeit entlang einem Strömungsgradienten über die Vorderseite des Trägers zu der vom Träger beabstandeten zweiten Öffnung fließt,
d) die zweite Probenkammer mindestens zwei Öffnungen aufweist, wobei durch eine erste Öffnung die Probenflüssigkeit aus der ersten Probenkammer entlang einem Strömungsgradienten über die Rückseite des Trägers und vorzugsweise in Gegenrichtung zur Strömung auf der Vorderseite des Trägers zu der zweiten Öffnung fließt,
e) die zweite Öffnung der ersten Probenkammer und die erste Öffnung der zweiten Probenkammer durch einen Kanal miteinander verbunden sind,
f) die zweite Öffnung der zweiten Probenkammer den Abfluss der Flüssigkeit ermöglicht,
g) die Freivolumina aus b.) zumindest teilweise mit einer Flüssigkeitssäule ausgebildet sind,
h) wobei der Strömungsgradient entlang der Vorder- und Rückseite des Trägers mittels Druckbeaufschlagung erfolgt und die Flüssigkeitssäule aus g.) mitführt.

Nachstehend das "erfindungsgemäße Verfahren".

Das erfindungsgemäße Verfahren erlaubt besonders vorteilhaft das reproduzierbare Beladen und Spülen eines Trägers mit Probenflüssigkeit zum Nachweis von Analyten. Hierbei wird eine qualitative Verbesserung durch die zeitweise Ausbildung einer Flüssigkeitssäule entlang dem Strömungsgradienten über den Träger und der relevanten Nachweisplätze mit den Rezeptormolekülen erhalten.

Erfindungsgemäß ist bevorzugt, dass der Träger die beiden Probenkammern trennt, so dass nahezu der gesamte Probenflüssigkeitsstrom zwischen den beiden Probenkammern durch den Träger oder durch den Kanal zwischen den Probenkammern verläuft, jedoch vorzugsweise nicht über die Ränder des Trägers hinweg. Dadurch ist gewährleistet, dass auch Träger mit variierenden Abmessungen in vergleichbarem Maß auf Ober- und Unterseite von der Probenflüssigkeit überströmt werden. Bevorzugt ist ebenfalls, dass eine etwaige zylindrische Wölbung des Trägers in Querrichtung, die von Beginn an oder erst nach Benetzung mit der Probenflüssigkeit besteht bzw. entsteht, durch die Befestigung des Trägers zwischen den Probenkammern verringert oder gar ausgeglichen wird. Auch dieser erfindungsgemäße Aspekt führt zu einer gewünschten gleichmäßigen und reproduzierbaren Umströmung des Trägers.

Erfindungsgemäß führt ein Kanal von der zweiten Öffnung einer ersten Probenkammer zur ersten Öffnung der zweiten Probenkammer. Dieser Kanal ermöglicht erst, die Probenkammern mit Hilfe des Trägers zu trennen und dennoch den Flüssigkeitsstrom von der ersten zur zweiten Probenkammer zu leiten, so dass Vorder- und Rückseite des Trägers gleichermaßen überströmt werden können. Überraschend ist, dass der Kanal die Spülwirkung nicht beeinflusst, obwohl dieser wie eine Drossel auf den Strom wirkt.

Ein weiterer erfindungsgemäßer Vorteil dieser Ausführungsform besteht darin, dass durch den Kanal ein Rücklauf von Probenflüssigkeit in Situationen verhindert wird, bei denen der auf die Probenkammern beaufschlagte Druck reduziert wird.

In einer bevorzugten Ausführungsform weist dieser Kanal von der zweiten Öffnung einer ersten Probenkammer zur ersten Öffnung der zweiten Probenkammer einen Durchmesser auf, der etwa dem der Probenzuführung und des Kanals für den Abfall (Waste) entspricht. Ferner ist bevorzugt, dass dieser Kanal im Durchmesser kleiner ist als der Durchmesser der Probenkammer bzw. des Freivolumens. Weiterhin ist bevorzugt, dass der Kanal ein Befestigungselement, insbesondere eine Querverstrebung, des Trägers umgibt und eine Rundbohrung aufweist.

Durch diese Aspekte ist gewährleistet, dass trotz Fertigungstoleranzen des Trägers reproduzierbar ein hinreichender Strömungsgradient bezüglich beider Trägeroberflächen, d.h. Vorder- und Rückseite entsteht, wobei vorteilhaft ein Transport eines wesentlichen Anteils, zumindest jedoch die Hälfte der Probenflüssigkeit außerhalb des Trägers erreicht werden kann. Dies gilt vor allem für jenen Bereich des Trägers, der beim Einströmen der Probenflüssigkeit zuerst erreicht oder benetzt wird und auf dem sich auf einem Nachweisplatz mindestens ein Rezeptormolekül befindet. Bevorzugt werden auch andere Lösungen, wie Spüllösung, im Wesentlichen außerhalb des Trägers transportiert.

Erfindungsgemäß bildet sich die Flüssigkeitssäule von der Vorder- oder Rückseite des Trägers zur gegenüberliegenden Kammerwand der jeweiligen Probenkammer(Kammerdeckel oder Kammerboden) aus.

In einer weiteren bevorzugten Ausführungsform beträgt der Abstand der ersten oder zweiten Oberfläche des Trägers zur Kammerwand 10 µm und mehr, wobei über der Vorderseite und / oder Rückseite eine Flüssigkeitssäule im Freivolumen steht, sobald der Strömungsgradient entsteht.

Bevorzugt ist jedoch ein Abstand von 10 bis 1.500 µm (1,5 mm) zwischen der Vorder- und Rückseite jeweils zur Kammerwand, wobei vorteilhaft kein Abreißen des Strömungsgradienten erfolgen kann, weiterhin bevorzugt ist ein Abstand von 80 µm bis 350 µm, und besonders bevorzugt ist ein Abstand von 120 µm bis 200 µm.

Weiterhin kann es bevorzugt sein, dass beide Abstände der jeweils Vorder- oder Rückseite des Trägers unterschiedlich sind und insbesondere beispielsweise 170 µm zum Kammerdeckel (Kammerwand, oben) und beispielsweise 150 µm zum Kammerboden (Kammerwand, unten) hin betragen. Die Variation ist abhängig vom gewünschten Strömungsgradienten in Abhängigkeit der Probenflüssigkeit.

Bevorzugt ist ferner, dass der Träger länglich zugeschnitten ist und an allen Seiten fest an der Probenkammer befestigt ist. Bevorzugt ist es, die Längsseiten zwischen zwei Rändern der Halbschalen, die eine Probenkammer und den Freiraum bzw. Freivolumen über und unter der Membran bilden, zu klemmen. Bevorzugt ist ferner, dass eine oder beide Stirnseiten des Trägers sich in einer Zwangsführung, beispielsweise einer Lasche befinden, um ein Verrutschen des Trägers während der Montage der Testkassette zu vermeiden und die Fertigung zu vereinfachen. Der Träger kann ebenfalls an den Längsseiten-oder Stirnseiten geklebt sein. Eine Kombination aus Klemmung und Klebung ist ebenfalls möglich. Weitere mögliche Verfahren zur Befestigung des Trägers umfassen das Eingießen der Ränder in einer Polymermatrix, Lösungsmittelkleben, Ultraschall- und Laser- und thermisches Schweißen, Lasermaskenschweißen, mechanische Fixierung wie beispielsweise Klemmen oder (Kunststoff-)nieten, oder 3d-Drucktechniken, bei denen eine Fixierung des Trägers durch die Formgebung erreicht wird.

In einer weiteren bevorzugten Ausführungsform der Erfindung enthält die Testvorrichtung neben den beiden Probenkammern eine dritte Kammer, die mit der zweiten Öffnung der zweiten Probenkammer fluidisch verbunden ist, und in der die zugegebenen Flüssigkeiten (Probenflüssigkeit, Spülflüssigkeit, Lösungen, etc.) gesammelt werden, nachdem sie durch die beiden Probenkammern geströmt sind. Diese dritte (Neben-)Kammer kann mit saugfähigem Material, beispielsweise Watte, Zellstoff, Polyacrylate oder funktionalisierte Polyacrylate in bekannter Weise ausgestattet sein. Bevorzugt ist ein Kammervolumen von mindestens 1,0 ml, insbesondere 5 - 10 ml.

In einer weiteren bevorzugten Ausführungsform ist die dritte Kammer durch mindestens eine Öffnung bzw. Entlüftung mit der Außenseite der Testkassette verbunden, so dass einer Druckbeaufschlagung der Testkassette ein Druckausgleich erfolgen kann. Besonders vorteilhaft ist, zwischen der dritten Kammer und der Außenseite der Testkassette noch eine zusätzliche vierte Kammer vorzusehen, damit Flüssigkeit, die aus der dritten Kammer austritt und beispielsweise aus einer Befüllung der Testkassette mit zu großen Flüssigkeitsvolumina oder aus einer zu schnellen Befüllung resultiert, in der Zusatzkammer gesammelt werden kann und nicht nach außen tritt, wo sie unerwünscht beispielsweise mit einem Anwender in Kontakt treten könnte.

Daher betrifft die Erfindung ebenfalls eine Ausführungsform, wobei der Abfluss aus der zweiten Probenkammer durch einen Kanal mit einer weiteren Kammer verbunden ist, wobei diese dritte Kammer
a) Teilweise ein saugfähiges Material enthält und
b) Eine Entlüftung enthält, die vorzugsweise als Kanal ausgebildet ist und besonders bevorzugt als Kanal mit mindestens einem Knick oder mindestens einer weiteren Kammer.

Das Austreten der Testflüssigkeit kann auch wie in PCT/EP2012/066155 (unveröffentlicht) offenbart durch geeignete halbdurchlässige Membranen in der weiteren Kammer verhindert werden, gleichwohl ist diese Ausführungsform jedoch deutlich aufwändiger in der Herstellung.

In einer weiteren bevorzugten Ausführungsform ist die dritte Kammer - der Abfallbehälter - durch einen Kanal mit der zweiten Probenkammer verbunden. Dadurch wird effektiv ein möglicher kapillarer Zug durch die Materialien im Abfallbehälter vermieden.

Ein bevorzugtes Verfahren zur Herstellung der Testkassette ist das Laserstrahlschweißen von Spritzgusskomponenten, mit dem eine ausreichende Präzision reproduzierbar erreicht werden kann. Überraschend wurde gefunden, dass ein Verfahren am besten geeignet ist, bei dem 3 Lagen strukturierte Spritzgusselemente - obere Halbschale, Mittelteil, untere Halbschale - verwendet werden, zwischen die vor dem Schweißen jeweils eine, teilweise mit Aussparungen oder Löchern versehene Folien gelegt werden. Die Fertigungskomplexität erscheint durch die beiden Folien zunächst größer und das Verfahren folglich ungünstiger. Überraschenderweise kann jedoch mit diesem Verfahren die Komplexität und zu erreichende Präzision der Spritzgussteile reduziert werden. Besonders bevorzugt ist eine Ausführungsform, bei der die Folien mit Aussparungen oder Laschen versehen sind, die die Fixierung der Träger vor der Verschweißung erleichtern, oder mit Löchern, um vertikale Kanäle zu ermöglichen.

Ein weiteres bevorzugtes Verfahren zur Herstellung der Testkassette ist das 3d-Drucken, das sich besonders für die Fertigung kleiner Stückzahlen eignet.

In einer weiteren bevorzugten Ausführungsform der Erfindung wird die Probenflüssigkeit nicht direkt auf den Träger appliziert, sondern über einen (Proben)Kanal in der Testvorrichtung in die Probenkammer mit Druckbeschlag eingegeben oder mittels Unterdruck eingesaugt. In einer bevorzugten Ausführungsform ist der (Proben)kanal parallel zum Träger ausgerichtet bzw. in Richtung des zu bildenden Strömungsgradienten.

In einer bevorzugten Ausführungsform befindet sich ein Diffusor am Eingang des ersten Freivolumens, der bewirkt, dass der Träger an den Rändern besser überströmt wird.

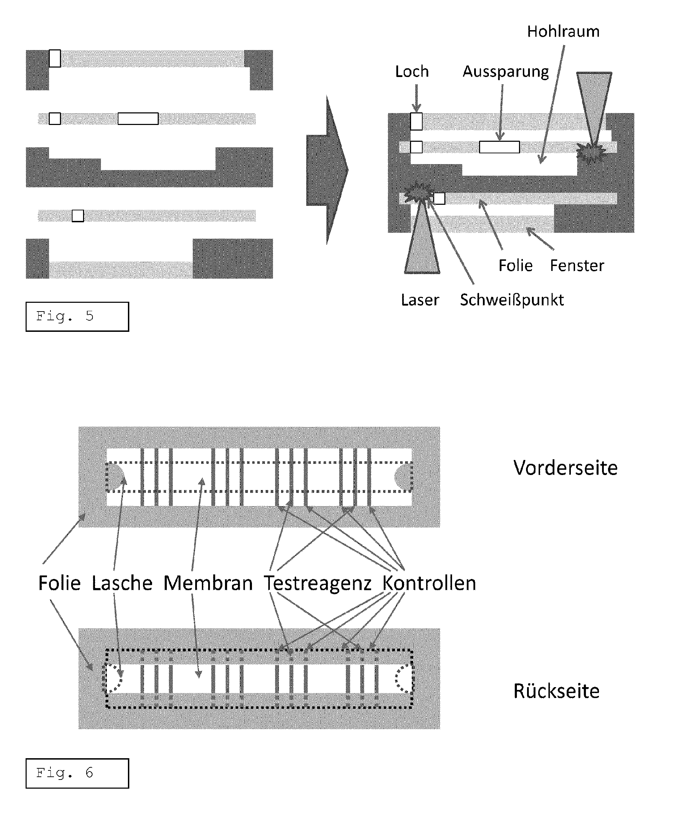

In einer weiteren bevorzugten Ausführungsform wird der Träger in der Weise an der Kammerwand befestigt, dass die vorhandene oder nach Benetzung mit einer Flüssigkeit entstehende Wölbung zur Oberseite zeigt (siehe Fig. 5).

Bevorzugt ist weiterhin die Verwendung von Probenvolumina oder Lösungsvolumina, die größer sind als das Flüssigkeitsvolumen, das der Träger aufnehmen kann, oder größer sind als das Gesamtvolumen des Trägers im feuchten Zustand (siehe Beispiele).

Der Strömungsgradient kann mittels einer Druckbeaufschlagung erzeugt werden, wobei die Probenflüssigkeit in die Probenkammer druckbeaufschlagt wird. Eine geeignete Druckbeaufschlagung kann manuell, insbesondere durch eine Spritze, Ampulle oder maschinell, insbesondere durch eine Pumpe oder einen Balg erzeugt werden.

Ein beispielhafter geeigneter Strömungsgradient kann wie folgt erreicht werden:

Eine ausreichende Druckbeaufschlagung ist zum Beispiel 150 hPa über der Kammer, bei einem typischen Probenflüssigkeitsvolumen von 95 µl, falls die Höhe des Freivolumens 320 µm, seine Breite 3,5 mm, seine Länge 50,5 mm und die angestrebte Befüllzeit 5s betragen. Bei einer zehntel Befüllzeit also 0,5s beträgt der notwendige Druck etwa 10-fach also 1.500 hPa unter sonst gleichen Bedingungen. Wird Unterdruck an der Auslassöffnung angelegt, beträgt die Befüllzeit mindestens 0,75s bei ansonsten gleichen Bedingungen.

Eine weitere bevorzugte mittlere Strömungsgeschwindigkeit der Probenflüssigkeit in der Kammer beträgt 0,1 m/s, die beispielsweise mit einer 1.000 µl Einmalspritze bei 4 Kanälen manuell bequem erreicht werden kann und einer Befülldauer von 5s entspricht (siehe Beispiel).

Weiterhin ist bevorzugt, dass zum Beispiel bei einer Probenflüssigkeit mit einem Volumen von 95 µl das Freivolumen über dem Träger eine Länge des Trägers von 50 mm und eine Breite von 3,5 mm aufweist, bei einer Gesamthöhe von 320 µm (Kammerboden zu Kammerdecke), wobei der Abstand der ersten Oberfläche zur Kammerdecke 170 µm beträgt und der Abstand der zweiten Oberfläche zum Kammerboden 150 µm beträgt.

Der maximale Über- oder Unterdruck und Fließwiderstand der Probenkammer ist ebenfalls bei anderen Dimensionen der Probenkammer und des Trägers in der Weise zu bemessen, dass eine Probenflüssigkeit die Auslassöffnung gemäß dem erfindungsgemäßen Verfahren in weniger als 5 Sekunden, vorzugsweise in weniger als 2 Sekunden erreicht.

Weiterhin ist vorteilhaft, dass der Durchmesser der Einlassöffnung bei einem gesetzten Probenflüssigkeitsvolumen von z.B. 95 µl 0,3 mm und der der Auslassöffnung 1,2 mm beträgt, wobei die Wegstrecke auf dem Träger bei gegebener Strömungsgeschwindigkeit vorzugsweise 50,5 mm beträgt. Andere Dimensionen sind entsprechend zu extrapolieren anhand der weiteren bevorzugten Ausführungsformen.

Daher betrifft die Erfindung in einer bevorzugten Ausführungsform eine optimierte Probenkammer bei einer Eingabe einer Probenflüssigkeit von 80 - 120 µl, wobei die Länge des Trägers 40 - 60 mm, die Breite des Trägers 2,0 - 5 mm, und der Einlassdurchmesser 0,15 mm bis 0,45, der Auslassdurchmesser 1 mm bis 1,5 mm, die Gesamthöhe der Probenkammer 260 µm bis 450 µm betragen.

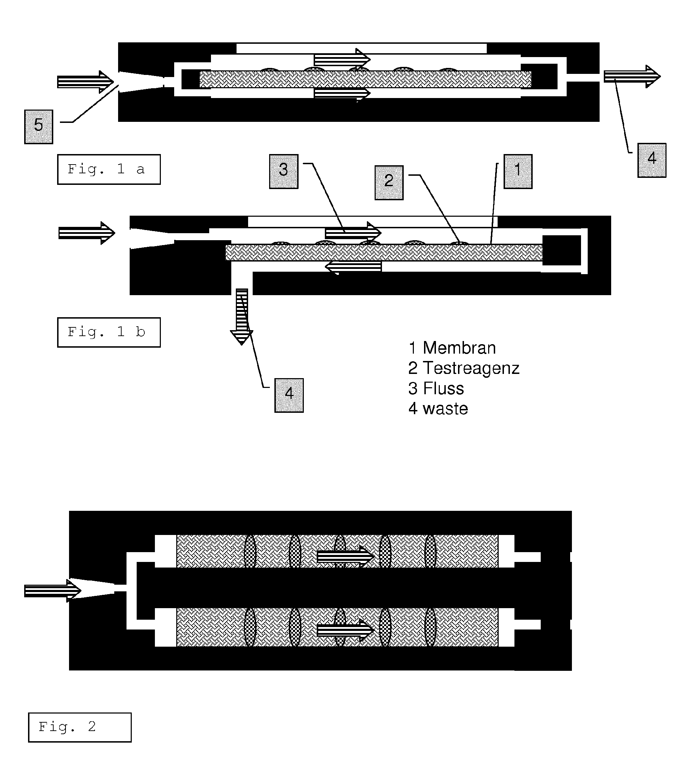

In weiteren Ausführungsformen kann der Strömungsgradient wahlweise a.) parallel an der ersten und zweiten Oberfläche des Trägers geführt werden oder b.) antiparallel an der ersten und zweiten Oberfläche des Trägers geführt werden (siehe Abb. 1a und 1b).

Besonders vorteilhaft ist eine Ausführungsform, bei der die Strömung zunächst entlang der Vorderseite des Trägers geleitet wird, durch einen weiteren Kanal von der Vorderseite des Trägers zur seiner Rückseite geleitet und folglich in Gegenrichtung zu einer weiteren Öffnung des zweiten Freivolumens (Abb. 1b) geleitet wird. Bei dieser Ausführungsform wird die Strömung nicht geteilt, sondern die Strömung wird entlang beider Seiten des Trägers geleitet. Es besteht ein geringes Risiko einer Strömungsinhomogenität an der Bifurkation. Zudem ist bei dieser Ausführungsform bei gleichem Volumen der Probenkammer die Strömungsgeschwindigkeit der Lösung doppelt so hoch, wie bei einer Ausführungsform mit gleichgerichteter Strömung. Bei gleichem Volumen an eingesetzter Flüssigkeit wird zudem aufgrund der höheren Strömungsgeschwindigkeit eine bessere Spülwirkung erreicht.

Erfindungsgemäß ist ein Testverfahren zum Nachweis mindestens einem Analyten aus einer Probenflüssigkeit vorgesehen, bestehend aus zwei Probenkammern, die vorzugsweise durch einen für eine Probenflüssigkeit teilweise durchlässigen Träger bestehend aus mindestens zwei Oberflächen getrennt werden, wobei eine Vorderseite des Trägers zur ersten Probenkammer und eine Rückseite des Trägers zur zweiten Probenkammer weist, und
a) mindestens ein Rezeptormolekül auf der Vorderseite eines Trägers fixiert ist,
b) über der Vorder- und Rückseite des Trägers jeweils zumindest ein Freivolumen ausgebildet ist, welches von einer Kammerwand begrenzt ist,
c) die Probenflüssigkeit über einen sich in zwei Kanäle verzweigenden Kanal, wobei jeder in eine der beiden Probenkammern mündet, in die Probenkammern verteilt wird, wobei durch jeweils eine erste Öffnung die Probenflüssigkeit entlang jeweils einem Strömungsgradienten über die Vorder-und Rückseite des Trägers zu jeweils einer vom Träger beabstandeten zweiten Öffnung fließt,
d) die jeweils zweite Öffnung der Probenkammern in mindestens einen Kanal übergehen, die den Abfluss aus den Probenkammern ermöglicht,
e) die Freivolumina aus b.) zumindest teilweise mit einer Flüssigkeitssäule ausgebildet sind,
f) wobei der Strömungsgradient entlang der Vorder-und Rückseite des Trägers mittels Druckbeaufschlagung erfolgt und die Flüssigkeitssäule aus e.) mitführt.

In einer weiteren bevorzugten Ausführungsform können mehrere Probenkammern (z.B. 1 bis 10) parallel geschaltet sein und aus einem Probenkanal gespeist werden (s. Abb. 2).

Daher betrifft die Erfindung ein Verfahren, wobei mehrere Probenkammern parallel aus einem Probenkanal mit mindestens einer Probenflüssigkeit versorgt werden.

Im Rahmen dieser Erfindung bedeutet der Begriff "Rezeptormolekül" Substanzen, wie Peptide, Proteine, Nukleinsäuren, Enzyme, Liganden, Rezeptoren, Antikörper oder Antigene, DNA, RNA, PNA, ebenfalls Moleküle nicht biochemischen Ursprungs, wie beispielsweise synthetische Moleküle. Die Testreagenzien können Kombinationen dieser Molekülsorten enthalten oder deren Fragmente, Konjugate, modifizierte, etwa glykolisierte oder phosphorylierte Formen. Lipide und Zucker sind ebenfalls erfindungsgemäß umfasst. Ferner können Naturstoffe und natürliche Extrakte in Testreagenzien vorkommen, wie dies bei Allergietests oder Nahrungsmittelunverträglichkeittests häufig der Fall ist. Mindestens ein Rezeptormolekül bindet mit mindestens einem Analyten aus der Probenflüssigkeit. Daher betrifft die Erfindung ebenfalls einen Bindungsassay, wobei mittels dem erfindungsgemäßen Verfahren besonders reproduzierbare Ergebnisse erhalten werden können.

Im Sinne dieser Erfindung besteht der Träger aus einem festen Material, aufweisend eine erste und zweite Oberfläche, ganz oder teilweise aus einer gelartigen, porösen, siebartigen, permeablen oder semipermeablen Membran, Dialysemembran, insbesondere einer beschichteten oder unbeschichteten Membran. Beispielsweise solche wie Nitrozellulose, PVDF, Zeolith, Sintermaterialien, insbesondere Polyethylenoxid, nachträglich mit Mikrokanälen versehenen Materialien wie Silizium, Glas oder Kunststoffen, die durch Laserbohren, Ionenbeschuß oder Ätzen durchlässig gemacht wurden. Der Träger kann beschichtet sein, beispielsweise mit Proteinen, insbesondere Antikörpern, Zuckern wie zum Beispiel Dextranen, oder aber mit Metallen, Glas, oder Kunststoffen, oder Carbonderivaten wie beispielsweise Carbon Nanotubes.

Im Sinne dieser Erfindung bedeutet "der Träger für eine Probenflüssigkeit teilweise durchlässig ist", dass die Probenflüssigkeit zumindest den Träger benetzt und eine teilweise Aufnahme der Probenflüssigkeit zumindest im Träger erfolgt. Ein geringes Durchtreten der Probenflüssigkeit ist ebenfalls nicht ausgeschlossen. Daher sind Membranen erfindungsgemäß bevorzugt und käuflich erhältlich.

Der Träger kann insbesondere zwei planparallele oder annähernd planparallele Oberflächen aufweisen, die erfindungsgemäß eine Vorder- und Rückseite ausbilden. Insbesondere kann der Träger die Form einer Membran oder eines flachen Balkens aufweisen. Die Träger können Nachweisplätze ausbilden und mit Rezeptormolekülen (Testreagenzien) versehen sein.

Ein oder mehrere Rezeptormoleküle sind auf der ersten Oberfläche des Trägers fixiert. Weiterhin kann mindestens ein Rezeptormolekül auf einer zweiten Oberfläche eines Trägers fixiert sein. Erfindungsgemäß können die Rezeptormoleküle auch in einer Schicht unterhalb der ersten oder zweiten Oberfläche fixiert sein. Die Rezeptormoleküle können zum Beispiel an oder nahe der Oberfläche eingetrocknet sein, entweder allein oder in einer stabilisierenden Matrix, oder als Lyophylisat aufgetragen werden und lösen sich bei Benetzung von der Oberfläche. Die Fixierung kann weiterhin durch Ausnutzung nicht kovalenter Wechselwirkungen erfolgen, wie sie beispielsweise zwischen Antikörper und Protein A bestehen, zwischen Biotin und Streptavidin, zwischen Hexa-Histidin und Nickel-NTA oder bei der Basenpaarung von DNA. Ferner kann ein Element der Rezeptormoleküle kovalent an die erste Oberfläche gebunden sein. In einer bevorzugten Ausführungsform bleibt das Rezeptormolekül bei Benetzung im Wesentlichen an der ersten Oberfläche fixiert, insbesondere zu mehr als 50%.

Eine Probenflüssigkeit im Sinne dieser Erfindung kann eine beliebige Substanz oder Substanzgemisch, ggfs. samt Lösungsmittel, beliebiger Herkunft sein, vorzugsweise jedoch eine biologische Flüssigkeit. Die Probenflüssigkeit kann insbesondere von einer Pflanze oder einem Tier stammen, insbesondere von einem Säugetier, insbesondere von einem Menschen. Die Probenflüssigkeit kann beispielsweise nicht abschließend sein: Vollblut, Halbblut, Serum, Speichel, Tränenflüssigkeit, Urin, Sekret, Hirnflüssigkeit oder prozessierte Formen solcher Flüssigkeiten oder solche Flüssigkeiten sein, die diese vorgenannten Flüssigkeiten enthalten. Die Probenflüssigkeit kann ebenfalls verdünnt vorliegen. Die Probenflüssigkeit enthält mindestens einen Analyten der an mindestens ein Rezeptormolekül adressiert ist oder eine nachweisbare Wechselwirkung verursacht.

Die Probenflüssigkeit kann weiterhin Reagenzien enthalten, wie etwa Liganden, Kompetitoren, Antikörper, insbesondere mit Digoxigenin konjugierte Antikörper, an die bis zu 100 Anti-Digoxigenen-Antikörper als Sekundärantikörper binden können und so eine Verstärkung des Signals bewirken, oder markierte Antikörper, Enzyme oder Enzymsubstrate, insbesondere solche, die einen Farbumschlag oder eine Lumineszenz bewirken, und kann eine zweite organische Probeflüssigkeit enthalten, die beispielsweise einen anderen Antikörper enthält und einen Farbumschlag in einer weiteren Farbe bewirkt.

Ferner kann die Probenflüssigkeit Stabilisatoren oder Antikoagulantien wie EDTA, Heparin oder Citrat enthalten.

Eine weitere Lösung kann ferner Detergenzien enthalten, Fängermoleküle zum Binden freier Substanzen, die nicht an ein Rezeptormolekül gebunden haben, oder Enzyminhibitoren, so dass die Lösung zum Spülen oder Abstoppen einer Reaktion geeignet ist.

Eine Lösung kann bidestilliertes, destilliertes oder Leitungswasser mit oder ohne weitere Substanzen enthalten, es kann zusätzlich einen Ionenaustauscher durchlaufen haben. Ferner kann sie übliche Pufferkomponenten zur Stabilisierung des pH-Werts oder zur Stabilisierung von Bestandteilen der Probenflüssigkeit enthalten.

Die Probenkammer ist ein Hohlraum, in dem sich der Träger befindet, und beide Öffnungen (Einlass, Auslass) enthält. Die Probenkammer kann eine für die manuelle Handhabung optimierte Form aufweisen, beispielsweise in Form eines flachen Quaders, der in einer Hand gehalten werden kann. Bevorzugt ist der Quader weniger als 1 cm dick. Die Flüssigkeiten können nacheinander mit Spritzen, die mit der anderen Hand bedient werden, in die Probenkammer eingespritzt werden. Die Oberseite der Probenkammer kann einen transparenten Deckel enthalten, der den Blick zumindest auf Teile des Trägers freigibt und eine schnelle visuelle Auswertung des Tests gemäß dem erfindungsgemäßen Verfahren ermöglicht. Ferner kann die Probenkammer in der Weise gestaltet sein, dass sie in ein Lesegerät gelegt werden kann, dass Vorrichtungen zum Befüllen der Probenkammer oder die Auslesung der Tests enthalten. Vorrichtungen dieser Art, insbesondere in miniaturisierter Form, können in einem weiteren Gehäuse oder dergleichen integriert sein.

Der Druck, mit dem die Probenflüssigkeit durch eine Öffnung in die Probenkammer fließt, kann vorzugsweise durch eine Spritze manuell erzeugt werden, wobei die Spritze bevorzugt ein Volumen aufweist, das nicht kleiner als ein Zehntel und nicht größer als das Zehnfache des Probenkammervolumens ist. Desweiteren ist bevorzugt, dass die Spritze einen Luer-Anschluss aufweist. Mit einer Spritze können die Flüssigkeiten zunächst aufgezogen und nachfolgend in die Probenkammer injiziert werden.

Die Spritze zur Druckerzeugung kann ebenfalls mit einer Spritzenpumpe betrieben werden. Vorteilhaft ist dabei, dass der Druck reproduzierbar und gleichförmig aufgebaut werden kann. Bei dieser Ausführungsform kann ein Schlauchverbinder zwischen Spritzenpumpe und der Probenkammer vorteilhaft sein, dessen kassettenseitiges Ende als Luer-Anschluss ausgestaltet sein kann. Weiterhin kann bevorzugt sein, ein Ventil mit mindestens zwei Wegen vorzusehen, so dass Reagenzien/Probenflüssigkeit aus einem oder mehreren Vorratsbehältern aufgezogen und nachfolgend in die Probenkammer abgegeben werden können.

Der Druck kann auch auf andere Weise manuell oder maschinell erzeugt werden, beispielsweise indem die Reagenzien/Probenflüssigkeit in einem kompressiblen und mit einer Öffnung versehenen Vorratsgefäß vorgehalten werden. Die Öffnung kann vorteilhaft mit einem Luer-Adapter versehen sein. Durch manuelle Druckausübung auf das Vorratsgefäß gelangt die Testlösung aus dem Vorratsbehälter in die Probenkammer. Der Druck auf den Vorratsbehälter kann auch maschinell erzeugt werden, beispielsweise indem ein Kolben, ein Hebel oder eine Quetschvorrichtung auf das Vorratsgefäß drückt, welches wiederum mit einem Motor, pneumatisch oder hydraulisch angetrieben werden kann.

Bei allen Verfahren und Vorrichtungen kann die Druckbeaufschlagung als Überdruck über der Einlassöffnung oder als Unterdruck über der Auslassöffnung anfallen.

Der Vorratsbehälter kann zur einmaligen Verwendung als Balg oder Blister ausgeführt sein, insbesondere in vorbefüllter und verschlossener Form. Vorteilhaft sind Bälge oder Blister aus Kunststoff oder Folie, insbesondere Einmalampullen. Es kann bevorzugt sein, Blister oder Einmalampullen in die Testkassette zu integrieren, so dass sie im gleichen Fertigungsgang gemeinsam hergestellt und die Vorratsbehälter in einem zweiten Schritt vorbefüllt werden.

In einer weiteren bevorzugten Ausführung der Erfindung wird eine Pumpe verwendet, die die Lösungen/Probenflüssigkeiten pumpt und in der Weise dem Probenraum zuführt. Die Pumpe kann sich zwischen Vorratsbehälter und Probenraum befinden und die Lösungen/Probenflüssigkeiten direkt pumpen, oder Luft oder eine Flüssigkeit in eine mit zwei Öffnungen versehenen Vorratsbehälter pumpen, so dass die darin befindliche Flüssigkeit verdrängt und in die Probenkammer gepumpt wird. Weiterhin kann vorgesehen sein, eine Pumpe zu verwenden, die vorwärts und rückwärts pumpen kann, und ein Ventil mit mindestens zwei Wegen zu verwenden, so dass Testreagenzien aus einem oder mehreren Vorratsbehältern herausgepumpt und nachfolgend in die Probenkammer abgegeben werden können. Die Pumpe kann sich auch hinter der Probenkammer befinden und Unterdruck in der Probenkammer erzeugen, so dass Flüssigkeiten aus einem kompressiblen Vorratsbehälter, der sich vor der Probenkammer befindet, durch Unterdruck in die Probenkammer gesogen und ggfs. weiter in den Abfallbehälter gesogen werden. Von besonderem Vorteil ist bei dieser Ausführungsform, dass die Pumpe mit keiner der Lösungen/Probenflüssigkeiten kontaminiert wird und daher ohne Reinigung weiter verwendet werden kann. Besonders geeignet sind Mikropumpen, wie beispielsweise O-run 100 oder O-run 200 von PARItec GmbH, Gräfelfing, Deutschland. Ferner sind Membranpumpen und Mikromembranpumpen, Schlauchpumpen und Zahnringpumpen besonders geeignet.

Eine Flüssigkeit, die eine Probenflüssigkeit oder eine Lösung sein kann, fließt in die Probenkammer mit einer Strömungskomponente parallel zur ersten und zweiten Oberfläche des Trägers. Befindet sich in der Flüssigkeit ein Analyt, der an mindestens einem Rezeptormolekül binden kann, so wird dies ermöglicht, sobald die Flüssigkeit mit dem Rezeptormolekül in Kontakt gebracht wird. Dabei kann es bevorzugt sein, nach dem Einbringen der Flüssigkeiten den Druck auf annähernd Atmosphärendruck zu entspannen, so dass die Flüssigkeiten auf dem Träger stehen, nicht oder kaum mehr strömen und Rezeptormolekül und Analyt inkubieren und binden können, ohne dass neue Flüssigkeit hinzugefügt werden muss. Ferner kann es bevorzugt sein, das Druckverhältnis eine Zeit lang umzukehren, so dass die Flüssigkeit sich in umgekehrter Richtung bewegt, oder die Druckverhältnisse eine Zeit lang zu alternieren, so dass die Flüssigkeit über dem Träger vor- und zurückbewegt wird. So ist im Vergleich zur unbewegten Flüssigkeit ein besserer Stoffaustausch zwischen Flüssigkeit und Träger möglich, ohne mehr Flüssigkeit zu benötigen.

Eine weitere Flüssigkeit/Lösung kann zum Spülen des Trägers verwendet werden. Auch beim Spülen kann es bevorzugt sein, die Flüssigkeit eine Zeit lang unbewegt oder schwach bewegt über dem Träger stehen zu lassen, um mit geringeren Flüssigkeitsmengen auszukommen. Ebenfalls beim Spülen kann der Druck alternierend beaufschlagt werden.

Überraschend ist, dass ein Testprotokoll wie das in Beispiel 1 beschriebene Resultate liefern kann, die mit dem bloßen Auge ähnlich gut auslesbar sind, wie beispielsweise aus dem Stand der Technik bekannten Lateral Flow Assays. Obwohl beim Lateral Flow Assay die Flüssigkeiten durch Kapillarkräfte durch den Träger und parallel zu seinen Oberflächen und folglich um die Rezeptormoleküle herum oder gar durch die Test-/Nachweisfelder hindurchfließen.

Ein Vorteil des erfindungsgemäßen Verfahrens ist weiterhin, dass es nicht unter Übersprechen leidet, das beim Lateral Flow Assay entstehen kann, wenn zwei auf unterschiedlichen Testfeldern befindliche Rezeptormoleküle an den gleichen Analyten binden.

Beim erfindungsgemäßen Verfahren geschieht das Überströmen des Trägers ausreichend schnell, sodass beim Kontakt mit der ersten Testreagenz keine wertverfälschende Depletion des Analyten aus der Flüssigkeit erfolgt und kein Einfluss auf die Wechselwirkung mit einem zweiten Rezeptormolekül auftritt.

Ein weiterer Vorteil des erfindungsgemäßen Verfahrens besteht darin, dass mehrere Flüssigkeiten nacheinander einfach, mit beliebigen zeitlichen Abständen und fast ohne Vermischung zugegeben werden können. Bei einem Lateral-Flow-Assay besteht die Möglichkeit dagegen ausschließlich mit Einschränkungen, da die Flüssigkeit lediglich mittels Kapillarwirkung des Teststreifens oder des Wickpads nachteilig bewegt wird. Einmal aufgetragen, bewegt sich eine Flüssigkeit kontinuierlich in Richtung des noch nicht benetzen Trägers oder Wickpads. Charakteristisch für die Kapillarwirkung ist weiterhin, dass gleichzeitig oder nacheinander auf einen Träger aufgetragene Flüssigkeiten ineinander laufen und sich vermischen. Infolge dessen können die sehr sensitiven enzymatischen Testverfahren, bei denen eine erwünschte Signalverstärkung beispielsweise über Meerrettichperoxidase oder alkalische Phosphatase eintritt, lediglich eingeschränkt durchgeführt werden, beispielsweise durch Eintrocknen. Tatsächlich beruhen kommerziell angebotene Lateral-Flow Tests ausschließlich auf Testprotokollen, bei denen kein enzymatischer Schritt vorkommt, und bei denen nicht mehr als ein Flüssigkeitsreagenz zur Probe zugegeben wird.

Auch gegenüber offen durchgeführten Tests, bei denen sich die Testlösungen im Überstand über einer Membran befinden, bestehen Vorteile. Bei diesen Tests wird eine freie oder in einem Rahmen fixierte Membran in einer Wanne manuell geschüttelt, wie es in EP1718970 der Anmelderin der Fall ist, oder die Wanne wird mit einem mechanischem Schüttler oder Taumelschüttler kontinuierlich bewegt. Durch das Schütteln wird eine Bewegung des Überstands relativ zur Membran induziert und folglich ein verbesserter Stoffaustausch für die Bindungsreaktion - wie auch für die Spülreaktion - erwirkt, der zu ähnlich guten Resultaten wie das erfindungsgemäße Verfahren führt. Die Vorteile des geschlossenen Aufbaus bestehen jedoch in der Vermeidung von Kontaminationen der Probe, dem Schutz des Anwenders vor Spritzern und Kontakt mit der Probeflüssigkeit, und der Unabhängigkeit von Betriebsparametern wie der Schüttelfrequenz des Schüttlers. Daher wird nach erfindungsgemäßen Verfahren die Reproduzierbarkeit verbessert

Überraschend ist ferner, dass die Resultate deutlich besser sind, wenn beide Oberflächen des Trägers mit den Flüssigkeiten in Kontakt kommen. Dies ist überraschend, da die zweite Oberfläche in beiden Fällen mit im Verhältnis ähnlichen Strömungen, Substanzmengen und Konzentrationen an Testflüssigkeit, Reagenzien und Spülflüssigkeiten in Kontakt kommt. Im experimentellen Vergleich wird jedoch eine deutlich schlechtere Spülwirkung beobachtet, wenn die zweite oder Rückseite des Trägers am Boden des Hohlraums befestigt ist.

Eine Auslesung des Testergebnisses kann dadurch erfolgen, dass die Testkassette einen transparenten Deckel aufweist, durch den die Testreagenzien beobachtet und Farbwert- oder Graustufenänderungen visuell ausgelesen werden können. Diese Anordnung ermöglicht auch eine fotometrische Auslesung des Testverfahrens, insbesondere bei Verwendung einer Optik, die das Testergebnis auf einen Bildsensor abbildet. Bevorzugt ist dieser Bildsensor ein CCD oder CMOS Sensor. Besonders kostengünstig und dennoch leistungsfähig sind Optiken aus dem Konsumerbereich, wie sie in Kameras, Webcams und Mobiltelefonen verwendet werden. Bei einem Test mit Farbumschlag oder Fluoreszenzänderung kann es bevorzugt sein, einen Farbfilter oder einen Farbsensor mit farbsensitiven Fotosensoren zu verwenden. Bei Verwendung von Fluoreszenz kann die Anregung der Fluoreszenz mit einer Lampe, einem Laser oder einer LED erfolgen.

In einer weiteren Ausführungsform werden elektrochemische Detektionsverfahren für die Auslesung verwendet, wie sie in den letzten Jahren allgemein für diagnostische Verfahren entwickelt wurden, so beispielsweise Dionex ED 40 Electrical Detector User Manual, http://www.dionex.com/en-us/webdocs/4529-34855-03.pdf, Gau, V. et al "Oral Fluid Nanosensor Test (OFNASET)with Advanced Electrochemical-Based Molecular Analysis Platform", Ann. N.Y. Acad. Sci. 1098: 401-410 (2007) doi: 10.1196/annals.1384.005. Wood, M. et al. "eSensor®: An Electrochemical Detection Based DNA Microarray Technology for Multiplexed Molecular Diagnostics" Abstracts of the 219th meeting of the Electrochemical Society (ECS) May 1, 2011 - May 6, 2011, Montreal, QC, Canada.

Daher betrifft die Erfindung ebenfalls einen Kit bestehend aus einem Testsystem mit zwei Probenkammern, die durch einen Träger mit mindestens zwei Oberflächen getrennt sind, bestehend aus einer Probenkammer enthaltend einen Träger samt fixierten Rezeptormolekülen zur Durchführung eines Bindungsassays mit einem Ein- und Auslass, wobei zur Kammerdecke und Kammerboden ein Freiraum besteht, ggfs. einer weiteren Nebenkammer, die mit der Probenkammer fluidisch verbunden ist, samt einem Mittel zur Druckbeaufschlagung und weitere Hilfs- und Zusatzstoffe, wie Stopplösung, Waschlösung etc. Weitere Ausgestaltungen können wie im vorstehenden Testsystem/Verfahren entsprechend vorgenommen werden.

Ein weiterer Gegenstand der Erfindung betrifft die Verwendung des erfindungsgemäßen Verfahrens oder eines Kit oder Testsystems (Vorrichtung) zum Nachweis von mindestens einem Analyten (Substanzen) aus einer Probenflüssigkeit bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmun- oder Stoffwechselerkrankungen, Infektionserkrankungen, Geschlechtserkrankungen, Nahrungsmittelunverträglichkeiten, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNA- oder RNA-Nachweis.

Besonders bevorzugt ist der Einsatz des erfindungsgemäßen Verfahrens oder eines Testsystems (Vorrichtung) im Point-of-Care Bereich.

### Beispiele und Figuren:

Diese Beispiele dienen ausschließlich zur Erläuterung der Erfindung, ohne die Erfindung auf diese Beispiele zu beschränken.

### Beispiel 1: Testprotokoll für Allergietest

### Vorbereitung

Testbox mit Röhrchen und Testkassette (Kit enthaltend Probenkammer) sowie die Patientenprobe auf Raumtemperatur (18-25°C) bringen.

Kurzzeitmesser, Einmalhandschuhe, Behälter für festen Abfall und Stift bereithalten.

### Testkassette und Spritze entnehmen.

Alle Inkubationen erfolgen bei Raumtemperatur.

Die in der Testbox (Kit) eingesteckten Reagenzröhrchen entweder vorab oder sukzessive im Testverlauf aufrecht in die entsprechend farblich markierten Haltevorrichtungen am oberen Rand der Box umstecken.

### 1.1. Probeneinbringung:

Proberöhrchen (roter Deckel) mit zuvor eingebrachter Probe öffnen und den Inhalt mittels der Spritze vollständig und blasenfrei in die Testkassette überführen. Dazu Spritze in die Probenöffnung stecken und Inhalt zügig einspritzen.

Testkassette auf ebener Unterlage ablegen und 4 Minuten inkubieren.

### 1.2. Zugabe der Testlösung:

Wie zuvor (Abschnitt 10.1.) beschrieben Inhalt der Konjugatlösung (gelber Deckel) mit Hilfe der Spritze in den Einlass der Testkassette zügig einspritzen und diese abgelegt 8 Minuten inkubieren.

### 1.3. Waschen:

Erstes Röhrchen mit Waschlösung (blauer Deckel) öffnen und Inhalt wie zuvor beschrieben in Einlass der Testkassette zügig einspritzen. Unmittelbar anschließend den Inhalt des zweiten Röhrchens mit Waschlösung (blauer Deckel) zügig einspritzen. Keine Inkubation erforderlich!

### 1.4. Entwicklung:

Erstes Röhrchen mit Farbsubstrat (weißer Deckel) öffnen und Inhalt in Einlass der Testkassette zügig einspritzen. Unmittelbar anschließend das zweite Röhrchen mit Farbsubstrat (weißer Deckel) öffnen, Inhalt blasenfrei in Einlass der Testkassette einspritzen und diese abgelegt 8 Minuten inkubieren.

Während der Entwicklung werden 2 parallele Referenzstriche unterschiedlicher Intensität und gegebenenfalls zusätzlich ein zentraler Teststrich in jedem Testfeld sichtbar.

### 1.5. Stoppen:

Röhrchen mit Stopplösung (grüner Deckel) öffnen und Inhalt in Einlass der Testkassette einspritzen. Hinweis: Die Testresultate sind für mindestens 12 Stunden stabil und können in dieser Zeit abgelesen werden.

### Beispiel 2: Testkammer (Probenkammer)

Im Folgenden werden Maße für die Membran und ihre Abstände zu anderen Elementen der Vorrichtung angegeben. Dabei ist zu beachten, dass alle Maße für den trockenen Zustand der Membran angegeben werden, und dass eine mögliche Durchbiegung der Membran oder Oberflächenunebenheiten oder ein eventueller Verzug der Spritzgussteile nicht berücksichtigt werden. Im feuchten Zustand kann die Membran quellen oder durchbiegen und so eine veränderte Dicke und veränderte Abstände zu anderen Bauelementen aufweisen.

Eine (verstärkte) Nitrozellulosemembran von 140 µm Dicke wird auf ein Maß von 50,5 mm * 6,3 mm zugeschnitten. Der resultierende Membranstreifen wird so auf eine mit einer Aussparung versehene Folie gelegt, dass der Streifen die Aussparung ganz abdeckt und an seinen Stirnseiten unter jeweils eine Lasche geklemmt wird, die sich an den stirnseitigen Enden der Aussparung in der Folie befinden. Die Folie wird mit dem Membranstreifen zwischen zwei spritzgegossene Halbschalen geklemmt, die mit der Folie und dem Membranstreifen beide Probenkammern gemäß Abb. 1b und 2 bilden, deren Breite 3,5 mm beträgt. Das Gesamtvolumen der Membran beträgt damit 44,5 µl, das der freien Membran 24,7 µl. Der Abstand der Membranoberseite zur der im Wesentlichen planaren Innenseite der oberen Kammerwand beträgt 170 µm, der von der Membranunterseite zur im Wesentlichen planaren unteren Kammerwand der Halbschale 150 µm. Das Volumen der oberen Kammer beträgt 30,04 µl, as der unteren 26,51 µl. Der Querschnitt des nicht geklemmten Teils der Membran beträgt 0,49 mm², der der Kammer 1,62 mm².

Die Kammer ist mit einer Einlassöffnung in der ersten Probenkammer in der Nähe eines Endes der Membran versehen, die einen Querschnitt von 0,3 mm² aufweist, und mit einer Auslassöffnung in der zweiten Probenkammer in der Nähe der Membran mit einem Querschnitt von 1,2 mm².

In der Testvorrichtung sind 4*2 Kammern parallel ausgeführt, so dass das gesamte Membranvolumen 98,98 µl beträgt, das der ersten Kammern ohne Zu- und Abläufe 120,19 µl und das der zweiten Kammern 106,05 µl. Sie werden gleichzeitig durch einen einzigen Zugang (Probenkanal) befüllt, welcher in 4 Kanäle auffächert, deren jeder mit einer ersten Kammer verbunden ist. Die Auslassöffnungen laufen wiederum zu einem einzigen Kanal zusammen, der mit einem in die Testvorrichtung integrierten Waste-Volumen verbunden ist, in dem saugfähiges Material untergebracht ist. Das saugfähige Material ist fluidisch mit keiner Membran verbunden, so dass die Saugwirkung sich auf freie Flüssigkeit beschränkt, die in den Waste gelangt. Die Testvorrichtung weist Fenster auf, durch die die mit Reagenz beladenen Bereiche der Teststreifen beobachtet werden können.

Auf die Testkassette ist eine opake und mit Aussparungen versehene Folie geklebt. Die Aussparungen dienen als Fenster, durch jedes dieser Fenster können die Messfelder für je ein Allergen betrachtet oder mit einem optischen Auslesegerät ausgelesen werden. Die Stege zwischen den Fenster, die sich entlang eines Trägers aufreihen, sind möglichst schmal gehalten, beispielsweise 2 mm oder weniger. Die Stege senkrecht dazu, von Träger zu Träger sind breiter, sodass sich auf ihnen ausreichend Platz für aufgedruckte Informationen zu den einzelnen Messfeldern befindet. Diese Informationen können Nummern, Buchstabenkürzel oder einen ganzen, beispielsweise das Allergen bezeichnenden Begriff, umfassen.

Bei einem Test wird in die Testvorrichtung nach Protokoll 1 folgende Substanzen und Mengen dazugegeben:

| | |
|---|---|
| 1) Probe einschließlich Probenverdünner | 380 µl |
| 2) Waschpuffer je Vorgang | 1.000 µl |
| 3) Substratlösung je Vorgang (2 mal Spülen) | 2*800 µl |
| 4) Antikörper / Konjugatlösung 1 | 800 µl |
| 5) Antikörper / Konjugatlösung 2 | 800 µl |
| 6) Stopp-Puffer | 1000 ul |

Das Volumen jeder einzelnen Testkomponente ist größer als das Volumen der Membranen (100 µl).

### Beschreibung der Abbildungen:

Fig. 1a: Längsschnitt durch eine Testkassette mit beiden Probenkammern, bei der Flüssigkeit an beiden Oberflächen des Trägers (1) - Vorder- und Rückseite - im Fluss (3) parallel zu einander geführt werden. Die Kassette wird durch die Einlassöffnung (5) druckbeaufschlagt, so dass die Flüssigkeit von dort zur Auslassöffnung und von dort durch einen weiteren Kanal zum Waste (4) (Nebenkammer (supra)) fließt.

Fig. 1b: Längsschnitt durch eine Probenkammer, bei der Flüssigkeit am Ende des Trägers (1) durch einen weiteren Kanal fließt und zur Rückseite des Trägers geleitete wird und an der zweiten Oberfläche (Rückseite) antiparallel zur ersten Oberfläche fließt.

Fig. 2: Aufsicht auf eine Probenkammer. Es können mehrere Träger parallel in einer Kassette angeordnet werden. Dazu kann der Fluss aufgeteilt werden oder die Träger befinden sich gemeinsam in einem Hohlraum.

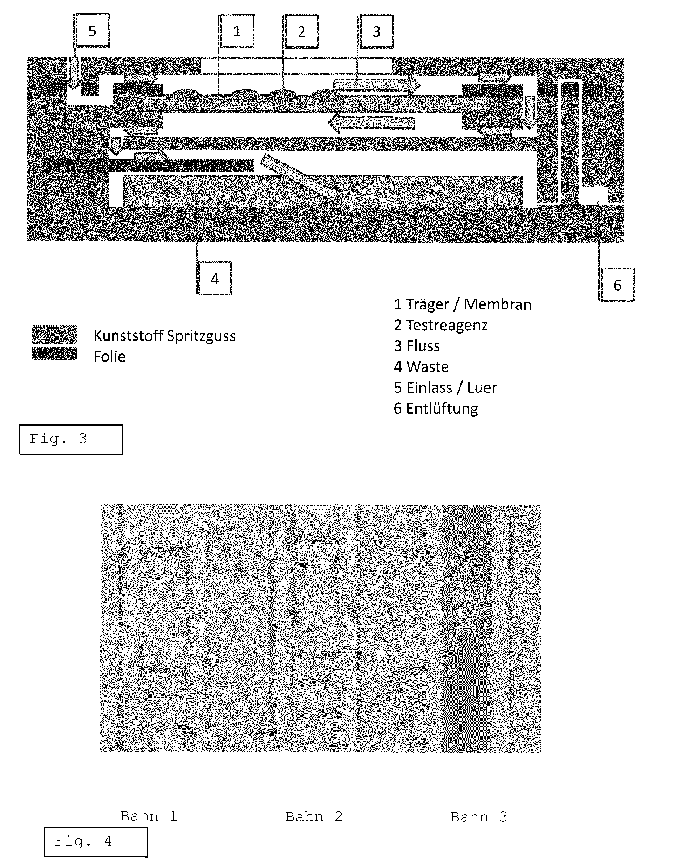

Fig. 3: Detaillierter Längsschnitt einer Ausführungsform, nicht maßstabsgetreu. Die Lösungen werden durch die Einlaßöffnung (5) eingeführt und fließen (3) über die Vorderseite des fixirten Trägers (1), auf dem sich Testreagenzien (2) befinden. Von dort gelangen die Lösungen über einen weiteren Kanal am anderen Ende des Trägers zur Rückseite des Trägers und fließen über diese zu einer Auslassöffnung. Ein zusätzlicher Kanal leitet die Flüssigkeiten zum Waste (4), der vorzugsweise ein saugfähiges Material enthält. Die Kammer für den Waste ist über eine Entlüftung mit der Außenwelt verbunden. Diese Entlüftung kann mit einer Schikanen und weiteren Kammern versehen sein, um ein Auslaufen des Waste zu verhindern.

Fig. 4: Immunoasssay mit drei identischen Membranen als Festphase, zeitgleich durchgeführt nach Protokoll aus Beispiel 1. In Bahnen 1 und 2 sind die Membranen von beiden Seiten umströmt, in Bahn 3 wurde nur die Oberseite überströmt. In allen Bahnen wurden identische Reagenzien und Volumina verwendet.

Fig. 5: Längsschnitt als Explosionszeichnung zur Verdeutlichung des geschichteten Aufbaus der Testkassette. Die Folien befinden sich zwischen jeweils einer in einigen Bereichen transparenten, spritzgegossenen Halbschale und einem ebenfalls spritzgegossenen Mittelteil. Der Laser verschweißt durch die Halbschalen hindurch Folie mit Halbschale und Mittelteil sowie Halbschalen und Mittelteil miteinander.

Fig. 6: Detaillierte Aufsicht auf den Träger, nicht maßstabsgetreu. Der Träger wird unter die stirnseitigen Laschen der Folie geklemmt und bedeckt die Aussparung in der Folie.

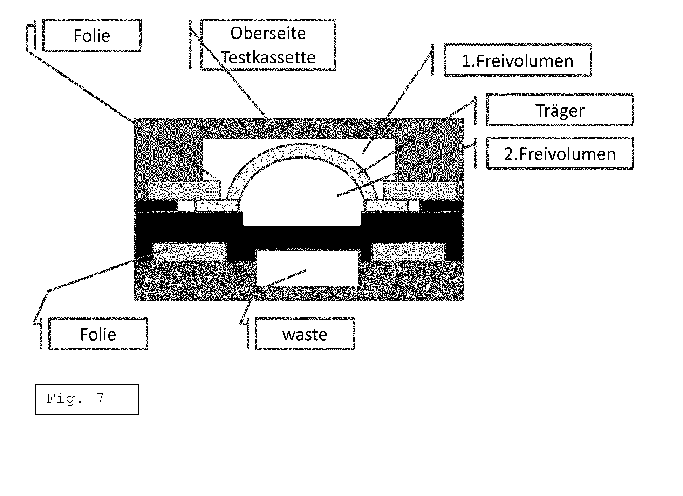

Fig. 7: Querschnitt durch eine Testkassette, nicht maßstabsgetreu. Der Träger ist an den Rändern der Probenkammern fixiert. Eine etwaige Wölbung des Trägers in Querrichtung ist stark übertrieben eingezeichnet. Sofern eine Wölbung vorhanden oder nach Benetzung entsteht, wird der Träger bevorzugt so eingelegt, dass die Wölbung zur Vorderseite hin ausgebildet ist.

## Patentansprüche

1. Testverfahren zum Nachweis von mindestens einem Analyten aus einer Probenflüssigkeit umfassend zwei Probenkammern, die durch einen Träger mit mindestens zwei Oberflächen getrennt sind, wobei eine Vorderseite des Trägers zur ersten Probenkammer und eine Rückseite des Trägers zur zweiten Probenkammer weist, und
a) mindestens ein Rezeptormolekül auf der Vorderseite eines Trägers fixiert ist,
b) über der Vorder- und Rückseite des Trägers jeweils zumindest ein Freivolumen ausgebildet ist, welches von einer Kammerwand begrenzt ist,
c) die erste Probenkammer mindestens zwei Öffnungen aufweist, wobei durch eine erste Öffnung eine Probenflüssigkeit entlang einem Strömungsgradienten über die Vorderseite des Trägers zu der vom Träger beabstandeten zweiten Öffnung fließt,
d) die zweite Probenkammer mindestens zwei Öffnungen aufweist, wobei durch eine erste Öffnung die Probenflüssigkeit aus der ersten Probenkammer entlang einem Strömungsgradienten über die Rückseite des Trägers und vorzugsweise in Gegenrichtung zur Strömung auf der Vorderseite des Trägers zu der zweiten Öffnung fließt,
e) die zweite Öffnung der ersten Probenkammer und die erste Öffnung der zweiten Probenkammer durch einen Kanal miteinander verbunden sind,
f) die zweite Öffnung der zweiten Probenkammer den Abfluss der Flüssigkeit ermöglicht,
g) die Freivolumina aus b.) zumindest teilweise mit einer Flüssigkeitssäule ausgebildet sind,
h) wobei der Strömungsgradient entlang der Vorder-und Rückseite des Trägers mittels Druckbeaufschlagung erfolgt und die Flüssigkeitssäule aus g.) mitführt.

2. Testverfahren zum Nachweis von mindestens einem Analyten aus einer Probenflüssigkeit, umfassend zwei Probenkammern, die durch einen Träger mit mindestens zwei Oberflächen getrennt sind, wobei eine Vorderseite des Trägers zur ersten Probenkammer und eine Rückseite des Trägers zur zweiten Probenkammer weist, und
a) mindestens ein Rezeptormolekül auf der Vorderseite eines Trägers fixiert ist,
b) über der Vorder- und Rückseite des Trägers jeweils zumindest ein Freivolumen ausgebildet ist, welches von einer Kammerwand begrenzt ist,
c) die Probenflüssigkeit über einen sich in zwei Kanäle verzweigenden Kanal, wobei jeder in eine der beiden Probenkammern mündet, in die Probenkammern verteilt wird, wobei durch jeweils eine erste Öffnung die Probenflüssigkeit entlang jeweils einem Strömungsgradienten über die Vorder-und Rückseite des Trägers zu jeweils einer vom Träger beabstandeten zweiten Öffnung fließt,
d) die jeweils zweite Öffnung der Probenkammern in mindestens einen Kanal übergehen, die den Abfluss aus den Probenkammern ermöglicht,
e) die Freivolumina aus b.) zumindest teilweise mit einer Flüssigkeitssäule ausgebildet sind,
f) wobei der Strömungsgradient entlang der Vorder-und Rückseite des Trägers mittels Druckbeaufschlagung erfolgt und die Flüssigkeitssäule aus e.) mitführt.

3. Testverfahren zum Nachweis von mindestens einem Analyten aus einer Probenflüssigkeit nach Anspruch 1 oder 2, umfassend zwei Probenkammern, die durch einen Träger mit mindestens zwei Oberflächen getrennt sind, wobei der Träger für eine Probenflüssigkeit teilweise durchlässig ist.

4. Testverfahren nach einem der vorstehenden Ansprüche, wobei der Kanal im Durchmesser kleiner ist als der Durchmesser der Probenkammer bzw. des Freivolumens.

5. Testverfahren nach einem der vorstehenden Ansprüche, wobei der Abfluss aus der zweiten Probenkammer durch einen Kanal mit einer weiteren Kammer verbunden ist, wobei diese dritte Kammer
a) Teilweise ein saugfähiges Material enthält und
b) Eine Entlüftung enthält, die vorzugsweise als Kanal ausgebildet ist und besonders bevorzugt als Kanal mit mindestens einem Knick oder mindestens einer weiteren Kammer.

6. Testverfahren nach einem der vorangehenden Ansprüche, wobei die Testkassette aus drei im Spritzguss gefertigten Bauteilen Halbschale - Mittelteil - Halbschale besteht, wobei sich zwischen mindestens einer Halbschale und dem Mitteilteil mindestens eine Folie befindet, die mit mindestens einer Aussparung und mindestens zwei Laschen zur Aufnahme von Trägern versehen ist, wobei besonders bevorzugt sich eine weitere Folie zwischen Mittelteil und der zweiten Halbschale befindet und diese Bauteile durch Laserschweißen und insbesondere durch Lasermaskenschweißen miteinander verbunden sind.

7. Testverfahren nach einem der vorangehenden Ansprüche, wobei der Abstand der Vorder oder Rückseite des Trägers zur Kammerwand 10 µm und mehr beträgt, insbesondere bis 1.500 µm.

8. Testverfahren nach Anspruch 7, wobei der Abstand 80 µm bis 350 µm, insbesondere 120 µm bis 200 µm, wobei ggfs. die Abstände der Vorder- und Rückseite des Trägers unterschiedlich sind.

9. Testverfahren nach einem der vorhergehenden Ansprüche, wobei der Träger aus einem festen Material besteht, ganz oder teilweise aus einer gelartigen, porösen, siebartigen, permeablen oder semipermeablen Membran, Dialysemembran, insbesondere einer beschichteten oder unbeschichteten Membran.

10. Testverfahren nach einem der vorhergehenden Ansprüche, wobei mindestens ein Rezeptormolekül auf einer zweiten Oberfläche eines Trägers fixiert ist.

11. Testverfahren nach einem der vorhergehenden Ansprüche, wobei der Strömungsgradient a.) parallel an der ersten und zweiten Oberfläche des Trägers geführt wird oder b.) antiparallel an der ersten und zweiten Oberfläche des Trägers geführt wird.

12. Testverfahren nach einem der vorhergehenden Ansprüche, wobei die Probenflüssigkeit eine biologische oder nichtbiologische Flüssigkeit ist, insbesondere Vollblut, Halbblut, Plasma, Serum, Speichel, Tränenflüssigkeit, Urin, Sekret, Hirnflüssigkeit oder prozessierte Formen solcher Flüssigkeiten, bakterienhaltige Lösungen, Wirkstoffe, insbesondere EDTA - stabilisierte Flüssigkeiten.

13. Testverfahren nach einem der vorhergehenden Ansprüche,
**dadurch gekennzeichnet, dass** die Druckbeaufschlagung mittels Über- oder Unterdruck erfolgt, insbesondere mit Hilfe einer Spritze, Ampulle, Pumpe oder Balg.

14. Testverfahren nach einem der vorhergehenden Ansprüche,
**dadurch gekennzeichnet, dass** bei einer Eingabe einer Probenflüssigkeit einschließlich 300 µl Probenverdünner von 340 - 540 µl, insbesondere 350 - 370 µl für Plasma und 480 - 520 µl für Blut als Probenflüssigkeit die Probenkammer, wie folgt gestaltet ist: Länge des Trägers 40 - 60 mm, Breite des Trägers 2,0 - 10 mm, Einlassdurchmesser 0,15 mm bis 0,45.

15. Testverfahren nach einem der vorhergehenden Ansprüche,
**dadurch gekennzeichnet, dass** mehrere Probenkammern parallel aus einem Probenkanal mit mindestens einer Probenflüssigkeit versorgt werden.

16. Testverfahren nach einem der vorhergehenden Ansprüche,
**dadurch gekennzeichnet, dass** Antikörper zum Nachweis des Analyten verwendet wird.

17. Kit bestehend aus einem Testsystem mit zwei Probenkammern, die durch einen Träger mit mindestens zwei Oberflächen getrennt sind, enthaltend einen Träger samt fixierten Rezeptormolekülen zur Durchführung eines Bindungsassays mit einem Ein- und Auslass, wobei zur Kammerdecke und Kammerboden ein Freiraum besteht, ggfs. einer weiteren Nebenkammer, die mit der Probenkammer fluidisch verbunden ist, samt einem Mittel zur Druckbeaufschlagung und weitere Hilfs- und Zusatzstoffe.

18. Verwendung eines Kits nach Anspruch 17 oder eines Verfahrens nach einem der Ansprüche 1 bis 16 zum Nachweis mindestens einem Analyten (Substanzen) aus einer Probenflüssigkeit bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmun-oder Stoffwechselerkrankungen, Infektionserkrankungen, Geschlechtserkrankungen, Nahrungsmittelunverträglichkeiten, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNA- oder RNA-Nachweis.
